# EUROPEAN PATENT SPECIFICATION

(11) **EP 3 378 475 B1**
(45) Date of publication and mention of the grant of the patent: **09.09.2020**
(21) Application number: 18162612.8
(22) Date of filing: 19.03.2018
(51) Int. Cl.: A61K 31/192, A61K 31/355, A61K 33/00, A61K 33/40, A61K 36/61, A61P 31/10, A61K 31/17, A61K 9/00, A61K 47/02, A61K 9/70, A61K 33/12, A61K 36/752, A61K 36/899, A61K 36/53

(54) **COMPOSITION AND KIT FOR THE USE IN THE PREVENTION OF RECURRENT ONYCHOMYCOSIS**
ZUSAMMENSETZUNG UND KIT ZUR VERWENDUNG IN DER PRÄVENTION VON WIEDERKEHRENDER ONYCHOMYKOSE
COMPOSITION ET KIT POUR UTILISATION DANS LA PRÉVENTION DE L'ONYCHOMYCOSE RÉCURRENTE

(30) Priority: 20.03.2017 IT 201700030541
(43) Date of publication of application: 26.09.2018
(73) Proprietor: Angeletti, Barbara, 61037 Mondolfo (PU) (IT); Livieri, Federica, 60019 Senigallia (AN) (IT)
(72) Inventor: LIVIERI, Federica, I-60019 Senigallia (AN) (IT); CAVALLO, Giovanni, I-00122 Ostia (RM) (IT); MORSIANI, Andrea, I-61032 Fano (PU) (IT)
(74) Representative: Sarpi, Maurizio

(56) References cited:
- WO-A1-2016/149376
- US-A1- 2014 079 804
- DATABASE WPI Week 201616 Thomson Scientific, London, GB; AN 2015-81081U XP002775459, & CN 105 078 793 A (UNIV SOUTH CHINA TECHNOLOGY) 25 November 2015 (2015-11-25)
- BUCK D S ET AL: "Comparison of two topical preparations for the treatment of onychomycosis: Melaleuca alternifolia (tea tree) oil and clotrimazole.", THE JOURNAL OF FAMILY PRACTICE JUN 1994, vol. 38, no. 6, June 1994 (1994-06), pages 601-605, XP009501433, ISSN: 0094-3509
- NIDHI AGGARWAL ET AL: "Preparation and Evaluation of Dermal Delivery System of Griseofulvin Containing Vitamin E-TPGS as Penetration Enhancer", AAPS PHARMSCITECH, SPRINGER NEW YORK LLC, US, vol. 13, no. 1, 1 December 2011 (2011-12-01), pages 67-74, XP035014941, ISSN: 1530-9932, DOI: 10.1208/S12249-011-9722-Y

## Description

### Field of application

In its most general aspect, the present invention relates to topical products for the reduction and elimination of fungal and microbial infections of the skin and of the nail, more particularly it concerns a composition for prevention of relapsing onychomycosis in a kit for the use in treatment of onychomycosis and the related method of use allowing a significant beneficial effect lasting over time on the nail of the patient.

### Background of the invention

Onychomycosis is the infection of the nail and the surrounding tissue supported by some fungi, molds and yeasts species, affecting around 30-50% of people over their lifetime; it is a difficult condition to be eradicated, therefore it is characterized by a high rate of recurrence, in the sense that very often the infection recurs periodically in the patient, after leaving the illusion of having been cleared; indeed the condition very frequently tends to become a chronic and recurrent disease.

Several factors are responsible for such relapse tendency. First of all, dermatophyte fungi and yeasts causing nail fungal infections are particularly resistant to treatments and require very long treatment times, even over 12 months. In order to eliminate them once and for all it is essential to follow the prescriptions indicated by the physician with precision and constancy, applying the topical remedies, and/or taking oral medications, every day, in the correct way and at the effective doses, for the entire expected period.

It is known that in the case of chronic conditions, or requiring very long treatment times, adherence to the treatment protocol (compliance) represents an issue of basic importance for the therapeutic efficacy of any treatment, since success depends on the effective compliance of the patient with the prescription. The direct consequence of non-compliance with treatment is the failure of treatment and the emergence of related clinical problems, including relapses, chronicity of the disease, with an increase in costs related to the ineffective management of the disease.

Although the onset of fungal nail infections does not represent a life-threatening condition, repeatedly forgetting the therapy or interrupting it sooner than necessary prevents "sterilizing" the nail and the surrounding tissues, making shortly new fungal colonizations very likely. Moreover, these oversights favor the selection of microorganisms less sensitive to antifungal drugs, which will therefore be more and more difficult to counteract during subsequent mycoses. Very often in association with not adequately treated fungal infections, secondary bacterial infections establish; the affected nails are subjected to deformation, hyperkeratosis, discoloration of the nail plate and fragility, so that, in addition to interfering with the normal function of the nail plate, fungal infections can cause pain, be unpleasant in appearance and involve a strongly negative psychosocial connotation (Chacon A. et al. Psycosocial impact of onychomycosis: a review Int J Dermatol, 2013, 52: 1300-7).

Also the type of treatment chosen, although apparently effective immediately, can contribute to expose to a greater risk of subsequent relapses, as intrinsically unable of neutralizing all the fungi present in the nail. This is the case, for example, of medicated enamels which, although having a good activity against the microorganisms causing onychomycosis, remain confined to the surface of the lamina and do not act sufficiently deeply on the responsible micro-organisms (fungi, molds and yeasts), hidden in the nail bed or in the surrounding tissues. Even though regularly applied for several months, then, in many cases they can offer only a temporary solution to the problem and do not exclude recurrences. Other factors promoting the return of mycoses are related to the patient habits of life.

After a first fungal infection while regularly practicing sports it is essential increasing attention to hygiene, such as washing and drying the feet carefully and wearing clean socks after each workout, replacing the usual shoes with a new pair to avoid re-infections and periodically use specific products for preventive purposes.

Some subjects, despite a strong commitment on the therapeutic front, in personal hygiene and in specific prevention, have periodically to reckon with recurrent onychomycosis. Mainly are subjects having skin and nails particularly predisposed to colonization by dermatophyte fungi or yeasts (first of all *Candida albicans*) or suffering from chronic diseases increasing this risk (reduction of immune defenses, psoriasis, diabetes etc.).

The US patent 7,258,872 describes a topically applied composition for treating infections caused by pathogenic organisms containing *Melaleuca alternifolia* oil extract (Tea tree oil), an immunostimulant agent strengthening the immune response of the tissue in the area of application and an antioxidant agent eliminating free radicals from infected tissue and inhibiting cellular damage thereby. The combination of these three ingredients in a suitable vehicle produces a composition enabling a non-specific treatment for most infections caused by pathogens. According to the invention, the immunostimulant agent is coenzyme Q10 and the antioxidant agent is vitamin E. However, no microbiological data concerning the eradication of the infection demonstrating the efficacy of the treatment based on the use of such composition are presented in the document. The U.S. Patent Application US 2003/0228382 describes a kit consisting of four compositions having antifungal, antimicrobial and antibacterial activity, each composition comprising the association of *Melaleuca alternifolia* oil extract (Tea tree oil) and *Australian eucalyptus* oil extract with carrier, emollients , surfactants, essential oils, fragrances and preserving agents, wherein the first of the four formulations is in the form of a washing solution further comprising ethanol, ethoxylated castor oil, and phenyl sulfonate zinc; the second of the four formulations is in the form of a spray further comprising vaseline, ethoxylated castor oil, jojoba oil, aloe vera and lanolin, glyceryl stearate, and octyl stearate; the third of the four formulations is a gel further comprising ethoxylated castor oil, at least one lauryl sulfate, coccabetaine and coconut diethanolamine; and the fourth formulation is in the form of balm further comprising vaseline, jojoba oil, aloe vera and lanolin. According to the Author, the formulations can be used individually, but their combined use, and in a certain order, ensures to obtain the best benefits, which however are never demonstrated in terms of abatement of the microbiological charge by appropriate and due laboratory analysis.

The Chinese patent CN 105 078 793 discloses a gel wash with antibacterial and anti-Candida activity comprising 6g of Tea tree oil, 100 mg of vitamin E TPGS and mandelic acid 0,5 g. Indeed, according to the description the antibacterial and anti-Candida gel has the following formulation: tea tree oil 2-10 %, chlorexidine acetate 0.5-8 %, watermiscible vitamin E derivant 0.01-1%, skin protectant 0.5-10%, surfactant 0.5-2%, alcohol material 5-30 %, carbomer 0.5-4 %, pH regulator 0.5-4 %, deionized water; moreover in the document is specified that the mandelic acid is counted as pH regulator in the group of several pH regulators all equally working and selectable by the skilled person, so that according to the description it is possible to think to replace mandelic acid with any pH regulator of the group. No specific biocide action or contribution against *Candida albicans* strain is attributed to the mandelic acid.

It is known that the oil extracted from the branches and leaves of the *Melaleuca alternifolia* has important medicinal properties including antiseptic, antifungal and antibacterial and is an increasingly present ingredient in cosmetic products. This essential oil is effective in many skin diseases such as acne, eczema, psoriasis and in many genitourinary infections such as chronic cystitis and vaginitis. The significant antifungal and antibacterial activity of the essential oil of Melaleuca is particularly evident against streptococci, fungi and Candida. Buck D.S. et al. in "Comparison of two topical preparation for the treatment of onychomycosis: Melaleuca alternifolia (tea tree) oil and clotrimazole". The Journal of family practice 199438: 601-605, discloses the antifungal activity of *Melaleuca alternifolia.*

An example of the use of Melaleuca oil extract in a topical application is described in U.S. Patent Application No. 5,894,020 wherein the oil is added to the ingredients of a soap in a concentration sufficient to treat the tinea pedis condition when the skin is washed with soap.

However, in relation to fungal infections causing onychomycosis there remains a strong risk of infection relapse and the difficulty in eradicating it, many topical compositions have been developed and used for treating fungal infections of the nails and surrounding skin have demonstrated very limited efficacy and some others, more aggressive, in an attempt to obtain greater efficacy, tend to be rather toxic, even for the living cells of the tissue surrounding the area of application, consequently, when applied locally, such antimicrobials and antifungals tend to cause damage and inflammation.

Therefore, it is strongly felt in the relevant technical field the need to have effective tools and methods for the treatment of onychomycosis not incurring in chronic recurrent infections, based on alternative compositions compared to those of the state of the art.

The present invention describes a composition using tea tree oil extract in association with mandelic acid and α-Tocopherol polyethylene glycol succinate for the prevention of recurrent onychomycosis in a kit for the use in the treatment of onychomycosis and the related method of use which ensures a significant beneficial effect on patient's fingernail, durable over time.

### Summary of the invention

The invention is defined by the claims. Any subject-matter falling outside the scope of the invention is provided for information purposes only. Furthermore, any references in the description to methods of treatment refer to the compounds, pharmaceutical compositions and medicaments of the present invention for use in a method for treatment of the human or animal body by theraphy.

The technical problem of the difficulty in eradicating fungal infections supporting onychomycosis and the risk of recurrence is solved by the present invention through a topical antifungal composition, characterized in that it includes the association of *Melaleuca alternifolia* oil extract, mandelic acid and α-Tocopherol polyethylene glycol succinate (in the present description this compound is also indicated with the synonyms: Vitamin E PEG succinate, Tocofersolan, Vit. E TPGS) and the method of treatment of the onychomycosis using it. Surprisingly, this composition was even more useful and advantageous when used in association with other topical compositions known and used for the treatment of fungal infections of the toenails and fingers of the hand, wherein it has shown a particular effectiveness in preventing or delaying the recurrence of the infection in subjects predisposed to the chronicity of the disease.

Therefore, a first object of the invention is the composition comprising the association of *Melaleuca alternifolia* oil extract (also known as tea tree oil), mandelic acid and α-Tocopherol polyethylene glycol succinate.

A second object of the invention is the use of such composition in the treatment and prevention of onychomycosis, in particular of recurrent forms of onychomycosis.

It has now surprisingly been found that the composition comprising the association of *Melaleuca alternifolia* oil extract, mandelic acid and α-Tocopherol polyethylene glycol succinate provides very good results in the treatment of recurrent fungal nail infections when used in association with another topical composition in form of gel, based on carbamide peroxide and laponite in an appropriate solvent system.

Therefore, a further object of the invention is the kit comprising the composition comprising the association of *Melaleuca alternifolia* oil extract, mandelic acid and α-Tocopherol polyethylene glycol succinate and another topical antifungal composition based on carbamide peroxide and Laponite, for the treatment of onychomycosis in the patients in the need thereof.

### Brief description of the drawings

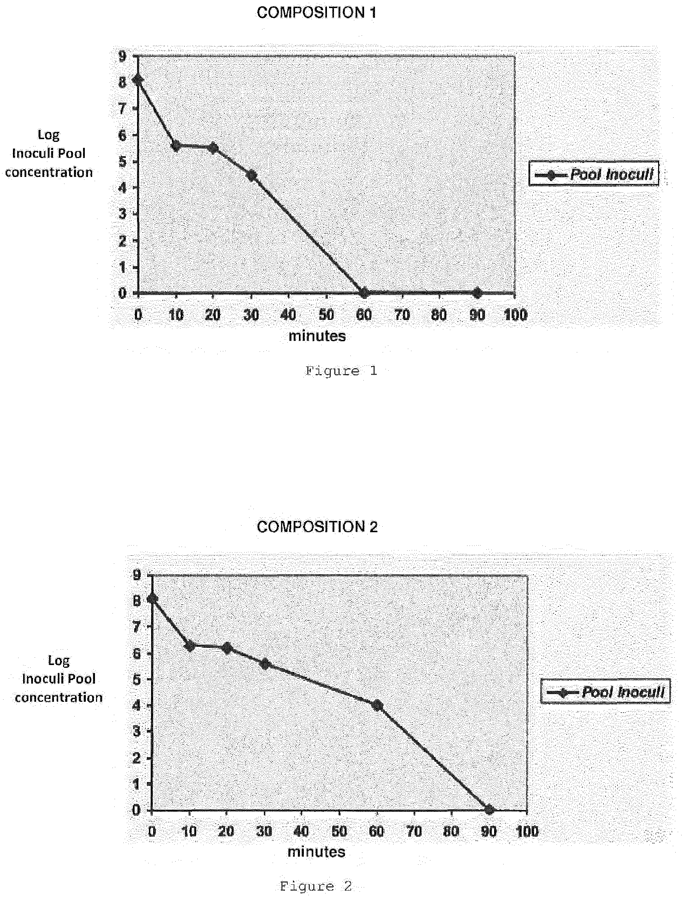

Figure 1 shows the logarithmic diagram of the inoculum pool concentration including dermatophyte fungal and *Candida albicans* strains in sample 1 comprising the association of *Melaleuca alternifolia* oil extract, mandelic acid and α-Tocopherol polyethylene glycol succinate.
Figure 2 shows the logarithmic diagram of the inoculum pool concentration including dermatophyte fungal and *Candida albicans* strains in sample 2 comprising *Melaleuca alternifolia* oil extract.

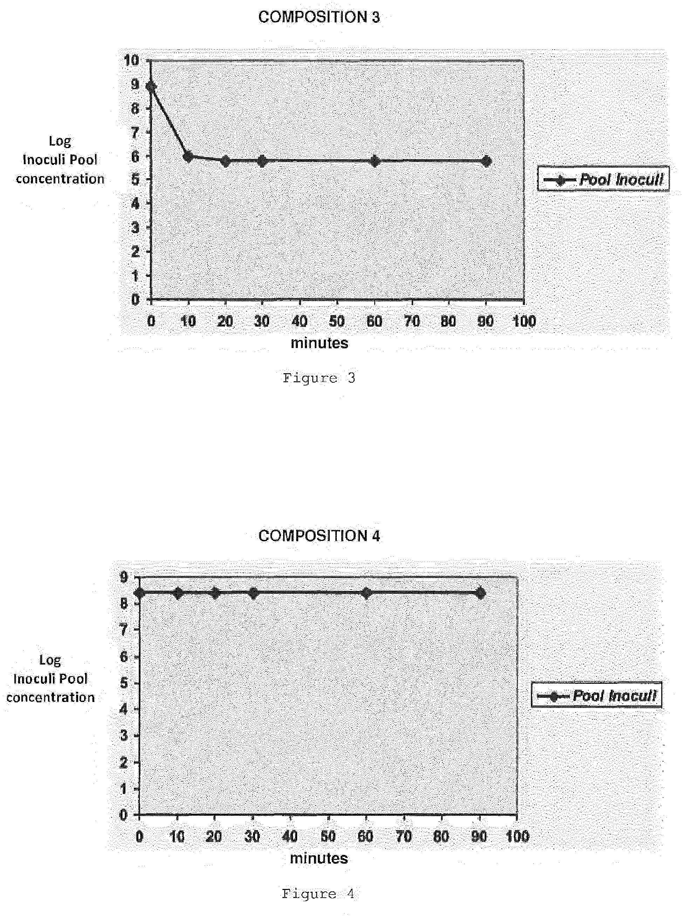

Figure 3 shows the logarithmic diagram of the inoculum pool concentration including dermatophyte fungal and *Candida albicans* strains in sample 3 comprising mandelic acid.
Figure 4 shows the logarithmic diagram of the inoculum pool concentration including dermatophyte fungal and *Candida albicans* strains in sample 4 comprising α-Tocopherol polyethylene glycol succinate.

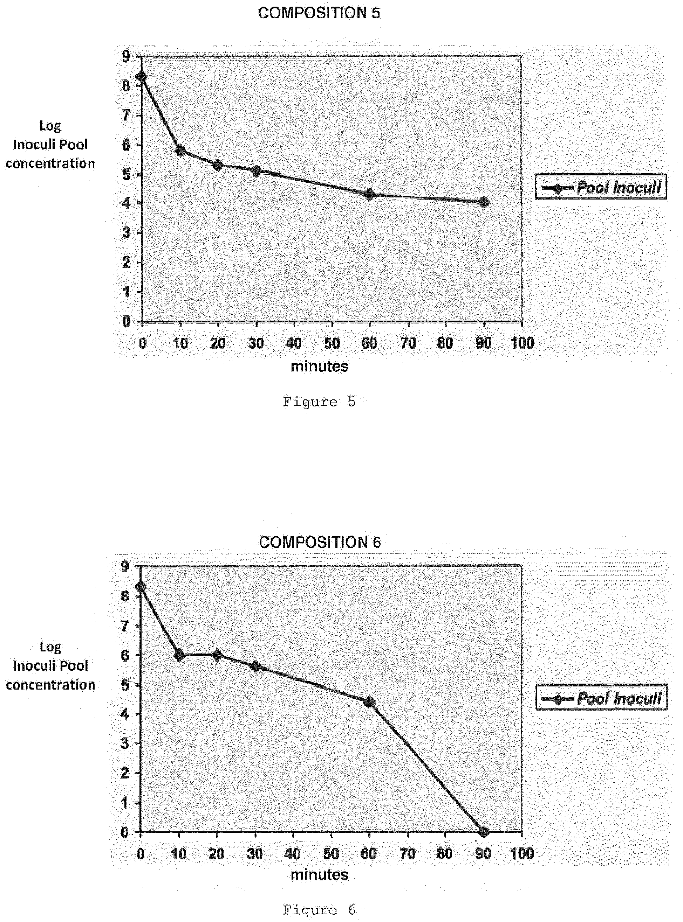

Figure 5 shows the logarithmic diagram of the inoculum pool concentration comprising dermatophyte fungal and *Candida albicans* strains in sample 5 comprising the association of *Melaleuca alternifolia* oil extract and mandelic acid.
Figure 6 shows the logarithmic diagram of the inoculum pool concentration including dermatophyte fungal and Candida albicans strains in sample 6 comprising the association of *Melaleuca alternifolia* oil extract and α-Tocopherol polyethylene glycol succinate.

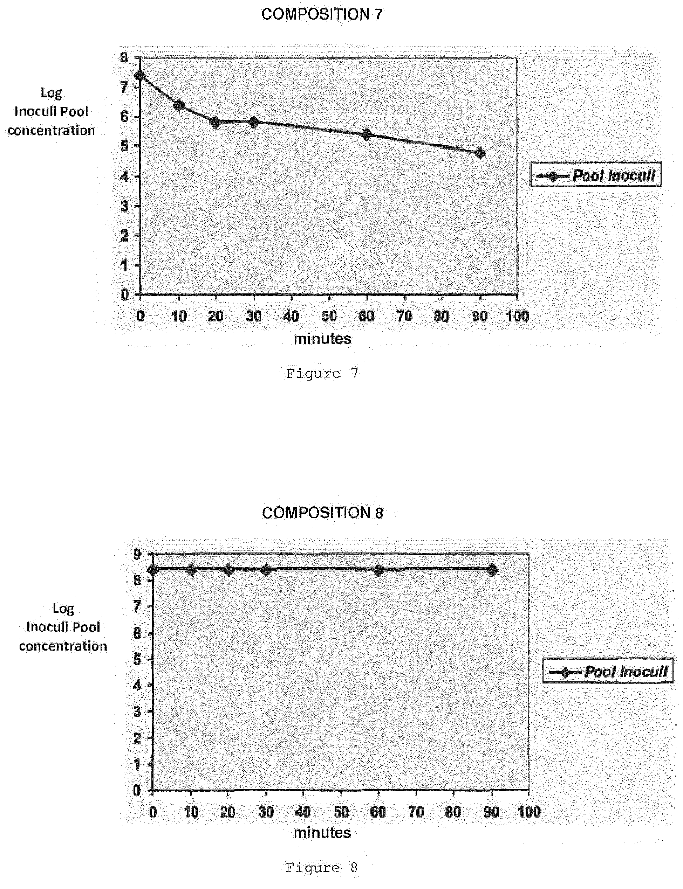

Figure 7 shows the logarithmic diagram of the inoculum pool concentration including dermatophyte fungal and *Candida albicans* strains in sample 7 comprising the association of mandelic acid and α-Tocopherol polyethylene glycol succinate combination.
Figure 8 shows the logarithmic diagram of the inoculum pool concentration including dermatophyte fungal and *Candida albicans* strains in sample 8 of the negative control, devoid of the association and its components.
Figure 9 shows the images of finger toes of patient of case study 1 at TO (A) and 224 days after the treatment starting (B).
Figure 10 shows the images of finger toes of patient of case study 2 at TO (A) and 224 days after the treatment starting (B).
Figure 11 shows the images of finger toes of patient of case study 3 at TO (A) and 224 days after the treatment starting (B).
Figure 12 shows the images of finger toes of patient of case study 4 at TO (A) and 224 days after the treatment starting (B).

### Detailed description of the invention

The aim of the present invention is to provide a topical composition for the treatment of onychomycosis comprising the association of:
a) *Melaleuca alternifolia* oil extract (tea tree oil),
b) mandelic acid,
c) α-Tocopherol polyethylene glycol succinate (Vit. E TPGS).

The antifungal and antibacterial properties of the *Melaleuca alternifolia* oil extract are known, it can be used to disinfect the skin, has a mild antibiotic effect for external use, counteracts acne, carbuncles and all common skin pimples, it is a bland remedy against fleas and lice infestations. Few drops of essential tea tree oil can be used directly on the skin, or diluted, as antifungal agent to combat skin fungus, nail fungus, athlete's foot phenomenon, however the administration of topical products containing tea tree oil, while causing a significant improvement in the appearance of the treated skin and nail, determines a temporary aesthetic effect, often within a few weeks/months the nail returns assuming the typical aspect of the mycotic nail, the nail appears thick, fragile, friable or jagged, deformed, opaque, yellowed or dark in color, sign of the onset of a new infection or the recurrence of the previous one. Therefore, the administration of topical products containing tea tree oil as an active ingredient, perhaps for its fungistatic activity, rather than fungicide, is not useful for the treatment and prevention of recurrent onychomycosis.

The mandelic acid, belonging to the category of alpha-hydroxy acids, is a compound widely used in cosmetics for the formulation of exfoliating, lightening and antioxidants creams; it is indicated as bacteriostatic and antiseptic agent, is a valid active ingredient for a wide range of skin problems: it treats acne and wrinkles, in adults it is also effective in the treatment of severe acne, produces excellent results in the treatment of inflammatory, comedonic and papular pustular acne; the administration of mandelic acid has proved to be effective even in cases of acne in which other types of medicines, or even antibiotics, have failed. The results may be visible as early as a few days after starting the treatment. It has been observed that a constant and gradual use of mandelic acid leads to significant improvements in the case of skin pigmentation abnormalities, the improvements have been recorded in particular for cases of: melasma, post-inflammatory hyperpigmentation and freckles. The mandelic acid also shares the same effectiveness of glycolic acid on wrinkles and fine lines of the skin. The result achieved lasts over time. One of the most important actions carried out by mandelic acid is the skin renewal: in addition to having an antibacterial action, the acid works on the skin breaking the link between dead and damaged cells still present on the skin and increasing the rate of cell regrowth.

The third ingredient of the association according to the present invention is α-Tocopherol polyethylene glycol succinate, a natural liposoluble form of vitamin E, rendered soluble in water by binding to a chemical agent called polyethylene glycol. This synthetic form of vitamin E has been developed to be more easily absorbed in the intestines of children having difficulty in absorbing fats and vitamin E from the diet, so increasing vitamin E levels in the blood and helping to prevent disorders of the nervous system due to vitamin E deficiency. In cosmetics and pharmaceuticals, tocofersolan is used, similarly to liposoluble natural vitamin E, for its antioxidant properties.

No antifungal and antibacterial properties have been reported in relation to vitamin E, nor to its water-soluble form, as also confirmed by the result of the experimentation conducted by the Applicants and presented below in the experimental section of this description.

Surprisingly it has been found that the *Melaleuca alternifolia* oil extract, in association with mandelic acid and α-Tocopherol polyethylene glycol succinate, in a topical composition comprising said association, exerts a much more effective and quicker antifungal action against a pool of microorganisms belonging to the species most frequently responsible for onychomycosis. In most cases (about 85 to 90%) the agent responsible for the onychomycosis is a dermatophyte keratinophile fungus that parasitize the keratinized tissues such as the horny layer of the epidermis and the skin appendages (hair, hair and nails in humans and feathers, feathers, hairs, hooves, etc. in animals); those mainly involved are *Trichophyton rubrum* and *Trichophyton mentagrophytes*; more rarely, the disease is due to yeasts and non-dermatophyte moldes. Surprisingly, the effect determined by the composition comprising the association of the three ingredients is improved and is established more rapidly than that determined by corresponding compositions comprising only one ingredient of the association, or their possible not complete combinations due to the absence of a component of the association itself. In the association according to the invention oil extract of *Melaleuca alternifolia*, mandelic acid and α-Tocopherol polyethylene glycol succinate are present respectively with a weight ratio: tea tree oil/mandelic acid/Vit. E TPGS of 12/0.5/0.5. In the preferred embodiments of the invention the association is in turn combined with carrier agents, solvents, essential oils, fragrances and perfumes, preservatives, emollient and moisturizing agents, antioxidant agents, vitamins, surfactants, suspending agents, binders, filmogens and viscosers, thickeners, chelating agents, buffering agents, pharmaceutically accepted and known to those skilled in the art, so as to provide a topical formulation of easy and pleasant use by the end user. Therefore, in some particularly preferred embodiments, the composition comprising the association according to the invention can further comprise essential oils such as lavender oil, bergamot, eucalyptus, geraniol, oil extracted from the germ and the inner film of the rice, hydrolyzed proteins of the wheat, lecithin, vitamins, such as ascorbyl palmitate, carnosine, lipoic acid, propylene glycol, thickeners such as xanthan gum, hydroxyethyl acrylate/sodium acryloyl dimethyl taurate copolymer.

An object of the invention is also the use of the described composition comprising the association of:
a) *Melaleuca alternifolia* oil extract,
b) mandelic acid,
c) α-Tocopherol polyethylene glycol succinate,
in the treatment of onychomycosis, and in particular of those forms of recurrent onychomycosis. Such use involves the application of an effective amount of the composition on the nail affected by the fungal infection and on the surrounding skin, once a day, generally in the evening before going to sleep, after cleansing; the composition is rubbed on nail and skin until completely absorbed. The treatment can also be prolonged in time since it is indicated as a useful remedy to counteract pathological conditions and recurring infections, which tend to chronicize, namely in particularly predisposed patients, and that due to toxicity problems cannot resort to systemic therapy.

The term "treatment", as used in the present description, means a reduction in the severity and/or frequency of symptoms, the elimination of symptoms and/or underlying cause, the prevention of the occurrence of symptoms and/or their cause, the improvement or remedy for the damage caused by the infection. Moreover, in a patient the use of the composition for the treatment allows the prevention of the onset of recurrent onychomycosis in a predisposed individual, therefore it determines a lengthening of the period of time elapsing between two fungal infection events in succession on the same nail. In fact, it is known that fungal infections can be established either as a primary disorder in a healthy nail, or settle secondarily in a nail already ill, traumatized, immunocompromised, predisposed or weakened also due to the effect of previous antifungal treatments. A common observation to those working in the sector is that a fingernail treated for a fungal infection is frequently recolonized by dermatophytes, non-dermatophytes or yeasts; generally, the therapeutic course of these patients ends up with a failure and the fungal infection becomes chronic.

With the expression "therapeutically effective amount" is meant a non-toxic amount, sufficient to provide the desired effect, i.e., the prevention or the treatment of nail fungal infection as above mentioned. Comprehensibly, the "therapeutically effective amount" will vary from subject to subject, depending on the general conditions thereof, modes of administration, and the like. Therefore, it is not always possible to specify the exact extent of the "therapeutically effective amount." However, an appropriate "effective" amount in any individual case may be determined by the operator podiatrist of ordinary skill in the art, that is the end user of the present invention. However, end user of the present invention can be also the patient in the need of the treatment against nail fungus infection who can topically administer the composition on its nail plate and bed at home according to the method of the invention.

The term "topical administration" is herein used according to its conventional meaning to indicate that the composition of the invention is applied on the nail plate of the hands and feet, and the surrounding areas, affected by the fungal infection.

According to the present invention the patient in need of treatment is a Mammal, preferably a Primate, most preferably belongs to Human species.

It has surprisingly been observed that the use of the composition according to the invention comprising the association of *Melaleuca alternifolia* oil extract, mandelic acid and Vit. E TPGS in combination with another topical composition used for the treatment of the onychomycosis allows to effectively treat the fungal infection also in that group of patients wherein the use of this other topical composition for the treatment of onychomycosis has not been able to eradicate the fungal infection, as evidenced by the microscopic and cultural examination of the nail which has never given a negative result after treatment, or has returned to be positive in a short time after the interruption of treatment or during the treatment itself, indication of a relapse that in some cases may be due to the same pathogen of the first infection and in some cases to a different species or to a phenomenon of resistance. In general, the composition according to the present invention has proved to be particularly effective in eradicating fungal infection and preventing the recurrent onset in subjects predisposed when used in combination with another topical composition, exploiting the properties of one or more active ingredients, other than the association of *Melaleuca alternifolia* oil extract, mandelic acid and Vit. E TPGS, and also used for the purpose of counteracting and/or treating nail infection supported by dermatophyte fungi and yeasts. In this way each of the two compositions, when appropriately applied, by exercising their action in a specific way, reach different targets against the pathogen, so together they provide a synergistic effect against the infection whose purpose is to continually create different environmental conditions, suitable to counteract the metabolism of the responsible agent, acting on different molecular mechanisms of the microorganism. In such way the conditions favoring the resistance phenomenon and the relapses in the host are contrasted.

In a particularly preferred embodiment of the invention the use of the antifungal composition comprising the association of *Melaleuca alternifolia* oil extract, mandelic acid and Vit. E TPGS is effective when in association with the administration of the topical composition described in the Italian patent n. 102015000053512, and relating the composition for the use in the treatment of the onychomycosis comprising:
a) carbamide peroxide in an amount varying between 22 and 3% by weight of composition,
b) laponite in powder in a variable amount between 18 and 2% by weight of composition,
c) solvent system,
wherein the amount of carbamide peroxide preferably ranges from 19% to 6%, and even more preferably it is equal to 18.3% of the total composition, preferably the amount of laponite varies between 8 and 16%, even more preferably it is equal to 13.5% of the total composition, and wherein, according to the invention, carbamide peroxide (a) and laponite powder (b) in the provided quantitative ratio are mixed to form a powdery mixture which immediately before the use is dissolved in a solvent system (c) which according to the invention may be: deionized water, propylene glycol, or preferably an aqueous solution consisting of deionized water and propylene glycol, where the proportions of water and propylene glycol may vary from 90/10 to 10-90 v/v. The composition may further reckon on the presence of catalase enzyme inhibitors, such as for example hydroxylamine, or its functional analogs such as 3-amino-1,2,4-triazole.

At the time of the use the composition is made up by mixing carbamide peroxide (a) and laponite (b) powders in the expected quantitative ratios to form a powdery mixture which, immediately before the administration on the affected nail plate and surrounding areas, is dissolved in the above described solvent system. The gel thereby formed is placed on the area affected by the infection for the foreseen time and then is removed by simple rinsing with water or physiological solution. The object of the present invention is also the method for treating fungal infections of the nail and the surrounding area of skin in a patient requiring such treatment exploiting the fungicidal properties of the topical composition comprising the association of *Melaleuca alternifolia* oil extract, mandelic acid and Vit. E TPGS in association with the administration of the gel composition for treating onychomycosis comprising carbamide peroxide and laponite in a solvent system, such as deionized water, propylene glycol, or preferably in a mixture of deionized water/propylene glycol (90/10 - 10-90 v/v).

According to the treatment method of the invention the application of a therapeutically effective amount of the composition comprising carbamide peroxide and laponite in a solvent system, as described, sufficient to cover the region affected by the infection once a week, for a time ranging from 10 to 30 minutes for at least four weeks is provided. In some cases, the weekly application isrepeated for up to six weeks, and additionally three weeks, after nail cutting and a thorough cleaning of the nail bed to remove all nail fragments.

According to the treatment method of the invention the daily topical application of a therapeutically effective amount of the composition comprising the association of *Melaleuca alternifolia* oil extract, mandelic acid and Vit. E TPGS, generally in the evening before to go to sleep after cleansing is also provided. The composition is rubbed on the nail and surrounding skin until completely absorbed. The daily topical application can be repeated until needed or until the total remission of the infection, as evidenced by clinical observation, by direct microscopic analysis of a sample of lamina or nail plate and by culture examination that excludes the presence of fungus or pathogenic yeast. Daily administration of the composition comprising the association of *Melaleuca alternifolia* oil extract, mandelic acid and Vit. E TPGS, may be useful if protracted over time, or even cyclically repeated for periods of about three months, alternating with periods of non-treatment, as prophylaxis in subjects particularly predisposed to recurring infection. The removal of the lamina can be manually performed in a relatively simple manner by a podiatrist operator since in addition to the typical nail hyperkeratosis, onycholysis, that is the detachment of the nail plate from its bed, is a typical manifestation of fungal infection. Alternatively, the avulsion can be performed mechanically by means of a podiatry mill, or in any other way known to the operators in the field. This feature is particularly preferred as it favors the removal of a possible source of re-infection, represented by the infected nail residue *in situ*, ensures a greater speed of healing and the re-growth of a healthy and normal nail.

Topical usage of the two compositions in association, as described, provides a synergistic effect enabling to eradicate the fungal infection even in those patients in whom the use of a single type of topical antifungal composition in general, and more in particular of the use of the only composition based on carbamide peroxide and laponite, has not had a completely satisfactory therapeutic effect, thus extending the therapeutic efficacy group of the invention, or lengthening the time between two infection events in subjects predisposed to recurrent infection. Thus the method of treating onychomycosis, and in particular recurrent forms of onychomycosis, which according to the invention involves the use in combination of the weekly administration of the composition based on carbamide peroxide and laponite in solvent and the daily administration of the composition comprising the association of *Melaleuca alternifolia* oil extract, mandelic acid and Vit. E TPGS, in a patient requiring such treatment comprises:
a) cutting of the nail and deep cleaning of the nail bed to remove the nail fragments;
b) weekly topical application of the composition in the form of gel prepared as follows:
   i) preparation of the gel by dissolving the mixture in powder of carbamide peroxide and XG Laponite in the solvent, and mixing carefully, using a spatula, if necessary;
   ii) application of the gel obtained in i) on the nail to be treated;
   iii) removal of the gel;
   iv) rinsing with plenty of physiological solution or water, and in concomitance
c) daily topical application of the composition comprising the association of *Melaleuca alternifolia* oil extract, mandelic acid and Vit. E TPGS.

However, it will not escape to those skilled in the art that the proposed treatment scheme does not limit the scope of the invention. In fact, given the high degree of variability of infection levels in patients, with varying degrees of involvement and extension to fingernails, and the varying individual predisposition of patients of probable immunogenic determination, the dosage regimen of the applied composition and the timing of administration of the method according to the invention, is established by the podiatrist according to the real needs of the subject receiving the treatment. This need is determined by clinical and biochemical analysis to which the subject is subjected before starting treatment and at regular intervals during treatment to verify the effectiveness of the method, the good tolerability of the subject and the lack of side effects, as described.

A further object of the invention is a kit which facilitates the implementation of the method of treatment of the onychomycosis which uses the association of the two described compositions.

According to the invention the kit includes:
(a) a sachet-like container of material impermeable to moisture and air containing the amount of carbamide peroxide required for administration;
(b) a sachet-like container of material impermeable to moisture and air containing the amount of XGL laponite, and possibly of catalase inhibitor, necessary for administration;
(c) a small vial-type container containing the amount of solvent system selected from deionized water, propylene glycol or the mixture of deionized water/ propylene glycol (90/10 - 10-90 v/v) required for administration;
(d) a spatula or wand in inert material to promote mixing of the contents of the two packets;
(e) a container containing the topical composition comprising the association of *Melaleuca alternifolia* oil extract, mandelic acid and Vit. E TPGS and pharmaceutically acceptable agents;
(f) leaflet with instructions for use.

The composition according to the invention comprising the association of:
a) *Melaleuca alternifolia* oil extract (tea tree oil),
b) mandelic acid,
c) α-Tocopherol polyethylene glycol succinate (Vit. E TPGS),
is now illustrated by means of formulation examples of particularly preferred embodiments of the invention, which do not have a limiting intent, wherein all the quantities of the components are expressed as a percentage by weight.

### EXAMPLE 1

| Ingredients | % w/w |
|---|---|
| *Melaleuca alternifolia* oil extract | 12 |
| Mandelic acid | 0.5 |
| Vit. E TPGS | 0.5 |
| *Lavandula angustifolia* oil | 6.0 |
| Propylene glycol | 2.5 |
| Xanthan gum | 2.0 |
| Linalool | 1.5 |
| Hydroxyethyl acrylate/sodium acryloyl dimethyl taurate copolymer. | 1.11 |
| Squalane | 0.765 |
| Carnosine | 0.5 |
| Lipoic acid | 0.5 |
| Limonene | 0.204 |
| Polysorbate 60 | 0.165 |
| Tetrasodium glutamate diacetate | 0.094 |
| Geraniol | 0.06 |
| Sorbitan isostearate | 0.045 |
| Citronellol | 0.006 |
| Coumarine | 0.006 |
| Deionized water a | Up to 100 |

### EXAMPLE 2

| Ingredients | % w/w |
|---|---|
| *Melaleuca alternifolia* oil extract | 12 |
| Mandelic acid | 0.5 |
| Vit. E TPGS | 0.5 |
| Essential oil of *Citrus bergamia* | 6.0 |
| Caprylic/capric triglyceride | 5.425 |
| Ethylhexyl palmitate | 5.037 |
| Glycerine | 4.65 |
| Cetylstearyl alcohol | 3.83 |
| Limonene | 2.84 |
| Steareth-2 | 2.8 |
| Steareth-20 | 1.4 |
| Propylene glycol | 2.5 |
| Tocopheryl acetate | 1.55 |
| Xanthan gum | 2.0 |
| Linalool | 0.9 |
| Dimethicone | 0.62 |
| Benzyl alcohol | 0.5 |
| Carnosine | 0.5 |
| Lipoic acid | 0.5 |
| Oil extracted from rice pulp kernels | 0.387 |
| Hydrolyzed wheat proteins | 0.11 |
| Sodium dehydroacetate | 0.11 |
| Lecithin | 0.087 |
| Tetrasodium glutamate diacetate | 0.05 |
| Dehydroacetic acid | 0.032 |
| Tocopherol | 0.014 |
| Ascorbyl palmitate | 0.010 |
| Citric acid | 0.003 |
| Deionized water | Up to 100 |

### Experimental part

### In vitro evaluation of antifungal activity against fungi dermatophytes and yeasts.

The activity of the composition according to the invention was evaluated with respect to four of the most responsible species for fungal infection of the nail, in particular, three strains of dermatophyte fungi: *Trichophyton rubrum, Trichophyton mentagrophytes* and *Epidermophyton floccosum* and yeast *Candida albicans,* by comparing the efficacy of action of eight embodiments of the composition according to example 1 differing in the presence of one or two out the three elements of the association. In this way the test allowed to assess the contribution of each member of the association of *Melaleuca alternifolia* oil extract (tea tree oil), mandelic acid, α-Tocopherol polyethylene glycol succinate, and the synergic effect of the three ingredients in the determination of antimicrobial activity against the tested species.

The activity was evaluated by means of viability test of the tested strains by counting the remaining vital colonies after incubation of dilutions of known titre of the four pooled strains in Sabouraud Dextrose Agar plates at 22-25 °C for 5 days.

| | |
|---|---|
| Tested strains: | *Trichophyton mentagrophytes* ATCC 9533 |
| | *Trichophyton rubrum* IHEM 02770, |
| | *Epidermophyton floccosum* ATCC 52066, |
| | *Candida albicans* ATCC 10231. |

### Tested compositions according to the invention:

Sample 1: formulation as described in Example 1. *Lavandula angustifolia* oil, Propylene glycol, Xanthan gum, Linalool, Hydroxyethyl acrylate / sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **the association of *Melaleuca alternifolia* oil extract, mandelic acid and α-Tocopherol polyethylene glycol succinate.**
Sample 2: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and ***Melaleuca alternifolia* oil extract.**
Sample 3: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **mandelic acid.**
Sample 4: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **α-Tocopherol polyethylene glycol succinate.**
Sample 5: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of *Melaleuca alternifolia* oil extract and mandelic acid.**
Sample 6: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of *Melaleuca alternifolia* oil extract and α-Tocopherol polyethylene glycol succinate.**
Sample 7: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of mandelic acid and α-Tocopherol polyethylene glycol succinate.**
Sample 8: (Negative Control) *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water.

Preparation of the strains. The freeze-dried strains obtained from the manufacturer have been cultured in their growth media and incubated at optimal temperature. Preparation of suspensions of the tested strains having known titre. According to the procedure provided in the Italian Official Pharmacopoeia (FUI XII ed.) a stock-suspension having known concentration for each single strains: *Trichophyton mentagrophytes, Trichophyton rubrum, Epidermophyton floccosum* and *Candida albicans,* grown in soil Tryptic Soy Broth (Liofilchem Srl) was preparared. The stock suspensions for each strain have then been used to prepare a stock suspension of the pool suspension of known title.

Procedure. The pool of strain was inoculated into four plates with optimal growth media each containing the composition (sample 1-8) to be assessed for its antifungal activity. Colture samples were withdrawn after 10, 20, 30, 60, 90 minutes according to F.U.I. XII ed.

### Results

### Sample 1 - composition with the association of Melaleuca alternifolia oil extract, mandelic acid and α-Tocopherol polyethylene glycol succinate (Example 1)

**Table 1 - Counts of living micro-organisms (cfu/ml)**

| | cfu/ml | Log |
|---|---|---|
| T0 (Initial pool concentration) | 13x10⁷ | 8.1 |
| T₁₀ₘ | 45x10⁴ | 5.6 |
| T₂₀ₘ | 31x10⁴ | 5.5 |
| T₃₀ₘ | 3x10⁴ | 4.5 |
| T₆₀ₘ | No recovery (NR) | NR |
| T₉₀ₘ | No recovery (NR) | NR |

**Table 2 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 2.5 | 2.6 | 3.6 | NR | NR |
| *Trichophyton rubrum IHEM 02770,* | | | | | |
| *Epidermophyton floccosum ATCC 52066,* | | | | | |
| *Candida albicans ATCC 10231* | | | | | |

The results show that sample 1, corresponding to the composition according to the invention comprising the **association of *Melaleuca alternifolia* oil extract, mandelic acid and α-Tocopherol polyethylene glycol succinate** has totally reduced the presence of microorganisms in the pool consisting of *Trichophyton mentagrophytes, Trichophyton rubrum, Epidermophyton floccosum, Candida albicans* after 60 minutes of treatment.

### Sample 2 - composition with Melaleuca alternifolia oil extract.

**Table 3 - Counts of living micro-organisms (cfu/ml)**

| | cfu/ml | Log |
|---|---|---|
| T0 (Initial pool concentration) | 13x10⁷ | 8.1 |
| T₁₀ₘ | 22x10⁵ | 6.3 |
| T₂₀ₘ | 17x10⁵ | 6.2 |
| T₃₀ₘ | 41x10⁴ | 5.6 |
| T₆₀ₘ | 97x10² | 4.0 |
| T₉₀ₘ | No recovery (NR) | NR |

**Table 4 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 1.8 | 1.9 | 2.5 | 4.1 | NR |
| *Trichophyton rubrum IHEM 02770,* | | | | | |
| *Epidermophyton floccosum ATCC 52066,* | | | | | |
| *Candida albicans ATCC 10231* | | | | | |

The results show that the sample 2 corresponding to the composition according to the invention comprising only ***Melaleuca alternifolia* oil extract,** has totally reduced the presence of microorganisms in the pool consisting of: *Trichophyton mentagrophytes, Trichophyton rubrum, Epidermophyton floccosum, Candida albicans* after 90 minutes of treatment.

### Sample 3 composition with mandelic acid

**Table 5 - Counts of living micro-organisms (cfu/ml)**

| | cfu/ml | Log |
|---|---|---|
| T0 (Initial pool concentration) | 7x10⁷ | 8.9 |
| T₁₀ₘ | 1x10⁶ | 6.0 |
| T₂₀ₘ | 7x10⁵ | 5.8 |
| T₃₀ₘ | 7x10⁵ | 5.8 |
| T₆₀ₘ | 7x10⁵ | 5.8 |
| T₉₀ₘ | 6x10⁵ | 5.8 |

**Table 6 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 2.9 | 3.1 | 3.1 | 3.1 | 3.1 |
| *Trichophyton rubrum IHEM 02770,* | | | | | |
| *Epidermophyton floccosum ATCC 52066,* | | | | | |
| *Candida albicans ATCC 10231* | | | | | |

The results show that the sample 3 corresponding to the composition according to the invention comprising mandelic acid has totally reduced the concentration of microorganisms in the pool consisting of *Trichophyton mentagrophytes, Trichophyton rubrum, Epidermophyton floccosum,* and *Candida albicans* of 2.9 logaritmic units after 10 minutes of treatment. After the composition has fungistatic activity up to 90 minutes.

### Sample 4 - composition with α-Tocopherol polyethylene glycol succinate

**Table 7 - Counts of living micro-organisms (cfu/ml)**

| | cfu/ml | Log |
|---|---|---|
| T0 (Initial pool concentration) | 26x10⁷ | 8.4 |
| T₁₀ₘ | 26x10⁷ | 8.4 |
| T₂₀ₘ | 26x10⁷ | 8.4 |
| T₃₀ₘ | 26x10⁷ | 8.4 |
| T₆₀ₘ | 25x10⁷ | 8.4 |
| T₉₀ₘ | 25x10⁷ | 8.4 |

**Table 8 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 0 | 0 | 0 | 0 | 0 |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that the sample 4 corresponding to the composition according to the invention comprising α-Tocopherol polyethylene glycol succinate has no fungicide activity.

### Sample 5 - composition with the association of Melaleuca alternifolia oil extract and mandelic acid

**Table 9 - Counts of living micro-organisms (cfu/ml)**

| | cfu/ml | Log |
|---|---|---|
| T0 (Initial pool concentration) | 20x10⁷ | 8.3 |
| T₁₀ₘ | 64x10⁴ | 5.8 |
| T₂₀ₘ | 19x10⁴ | 5.3 |
| T₃₀ₘ | 12x10⁴ | 5.1 |
| T₆₀ₘ | 2x10⁴ | 4.3 |
| T₉₀ₘ | 1x10⁴ | 4.0 |

**Table 10 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 2.5 | 3.0 | 3.2 | 4.0 | 4.3 |
| *Trichophyton rubrum IHEM 02770,* | | | | | |
| *Epidermophyton floccosum ATCC 52066,* | | | | | |
| *Candida albicans ATCC 10231* | | | | | |

The results show that sample 5, corresponding to the composition according to the invention comprising the **association of *Melaleuca alternifolia* oil extract and mandelic acid** has reduced the presence of microorganisms in the pool consisting of *Trichophyton mentagrophytes, Trichophyton rubrum, Epidermophyton floccosum, Candida albicans* of 2.5 logaritmic units, afterwards a gradual decrease up to 90 minutes treatment occurs.

### Sample 6 - composition with the association of Melaleuca alternifolia oil extract and α-Tocopherol polyethylene glycol succinate

**Table 11 - Counts of living micro-organisms (cfu/ml)**

| | cfu/ml | Log |
|---|---|---|
| T0 (Initial pool concentration) | 20x10⁷ | 8.3 |
| T₁₀ₘ | 2x10⁶ | 6.0 |
| T₂₀ₘ | 1x10⁶ | 6.0 |
| T₃₀ₘ | 6x10⁵ | 5.6 |
| T₆₀ₘ | 3x10⁴ | 4.4 |
| T₉₀ₘ | NR | NR |

**Table 12 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 2.3 | 2.3 | 2.7 | 3.9 | NR |
| *Trichophyton rubrum IHEM 02770,* | | | | | |
| *Epidermophyton floccosum ATCC 52066,* | | | | | |
| *Candida albicans ATCC 10231* | | | | | |

The results show that sample 6, corresponding to the composition according to the invention comprising the **association of *Melaleuca alternifolia* oil extract and α-Tocopherol polyethylene glycol succinate** has totally reduced the presence of microorganisms in the pool consisting of *Trichophyton mentagrophytes, Trichophyton rubrum, Epidermophyton floccosum, Candida albicans* 90 minutes after treatment.

### Sample 7 - composition with the association of mandelic acid and α-Tocopherol polyethylene glycol succinate

**Table 13 - Counts of living micro-organisms (cfu/ml)**

| | cfu/ml | Log |
|---|---|---|
| T0 (Initial pool concentration) | 23x10⁶ | 7.4 |
| T₁₀ₘ | 23x10⁵ | 6.4 |
| T₂₀ₘ | 67x10⁴ | 5.8 |
| T₃₀ₘ | 67x10⁴ | 5.8 |
| T₆₀ₘ | 26x10⁴ | 5.4 |
| T₉₀ₘ | 7x10⁴ | 4.8 |

**Table 14 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 1.0 | 1.6 | 1.6 | 2.0 | 2.6 |
| *Trichophyton rubrum IHEM 02770,* | | | | | |
| *Epidermophyton floccosum ATCC 52066,* | | | | | |
| *Candida albicans ATCC 10231* | | | | | |

The results show that sample 7, corresponding to the composition according to the invention comprising the **association of mandelic acid and α-Tocopherol polyethylene glycol succinate** has reduced of one logartimic unit the concentration of microorganisms in the pool consisting of: *Trichophyton mentagrophytes, Trichophyton rubrum, Epidermophyton floccosum, Candida albicans* and progressive gradual concentration reduction up to 90 minutes.

### Sample 8 - negative control composition without association components

**Table 15 - Counts of living micro-organisms (cfu/ml)**

| | cfu/ml | Log |
|---|---|---|
| T0 (Initial pool concentration) | 25x10⁷ | 8.3 |
| T₁₀ₘ | 25x10⁷ | 8.3 |
| T₂₀ₘ | 25x10⁷ | 8.3 |
| T₃₀ₘ | 25x10⁷ | 8.3 |
| T₆₀ₘ | 25x10⁷ | 8.3 |
| T₉₀ₘ | 25x10⁷ | 8.3 |

**Table 16 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533* | 0 | 0 | 0 | 0 | 0 |
| *Trichophyton rubrum IHEM 02770,* | | | | | |
| *Epidermophyton floccosum ATCC 52066,* | | | | | |
| *Candida albicans ATCC 10231* | | | | | |

The results show that sample 8 corresponding to the composition according to the invention without any association components has no fungicide activity.

The obtained results demonstrate that the association according to the invention is able to perform an *in vitro* very rapid fungicidal action against the species most responsible for the infection such as dermatophyte fungi: *Trichophyton rubrum, Trichophyton mentagrophytes*, *Epidermophyton floccosum* and *Candida albicans* yeast. Otherwise, the ingredients of the association, respectively oil extract of *Melaleuca alternifolia*, mandelic acid and α-Tocopherol polyethylene glycol succinate, when individually tested or have no antifungal activity, neither fungicidal, nor fungistatic, as in the case of α-Tocopherol polyethylene glycol succinate (composition 4), or have a limited ability to reduce the concentration of the inoculated pool which is established in the first ten minutes after inoculation, and subsequently presents a constant fungistatic activity over time which does not lead to the total elimination of the infection causative agent. In the case of mandelic acid (composition 3), or, as in the case of the oil extract of *Melaleuca alternifolia* (composition 2), after the initial fungicidal effect in the first ten minutes after the inoculum to the fungistatic action is accompanied by a very slow fungicidal effect which leads to an increase of 50% of the time necessary to have the total reduction of the concentration of the inoculum (90 minutes) compared to the time when the same effect is established on the same species with the association of the three compounds (60 minutes) (composition 1).

A further interesting observation on the synergistic effect of the ingredients of the association object of the invention is that when the α-Tocopherol polyethylene glycol succinate, without any antifungal activity itself, is added respectively to the composition comprising tea tree oil (composition 6) or to the composition comprising mandelic acid (composition 3) the fungicidal action does not substantially change, as shown by the logarithmic diagram profiles of the concentration of microorganisms in the inoculum pool (sample 2 vs. sample 6; sample 3 vs. sample 7). When, instead, the α-Tocopherol polyethylene glycol succinate as the third ingredient is added to the combination of tea tree oil and mandelic acid (composition 5) with fungistatic activity, it is observed the establishment of a very intense and rapid fungicidal action (sample 1 vs. sample 5), with a rapidity such as to reduce by 50% the total reduction time of the concentration of the inoculum pool, likely due to the action of tea tree oil (sample 1 vs. sample 2 and sample 6).

### Clinical study

An evaluation of the effectiveness of the method according to the invention based on the use of the association of the two described compositions was carried out on patients having history of recurrent onychomichosis with clinically and microbiologically proven infection due to dermatophytes and non-dermatophyte fungi. In fact, the diagnosis of cutaneous fungal infection in patients enrolled in the study was placed when there were clinical signs compatible, tested microscopy and microbial growing in the culture. The clinical diagnosis of onychomycosis was considered confirmed in the presence of positivity at microscopic examination of the nail samples and of the culture, the two latter considered essential for the diagnostic confirmation, in consideration of the frequency of false negative (Arrese J.E. et al. "Facing up to the diagnostic and management of uncertainty onychomycoses". Int. J. Dermatol 1999; 38, suppl.2, 1-6).

Patients enrolled in the study received the treatment according to the administration and dosing regimen provided by the method described herein. *Inclusion study criteria*: toenail and finger nail fungal infection in any of four clinical types of fungal nail infection: total dystrophic form, distal subungual onychomycosis, proximal onychomycosis and endonyx onychomychosis. Ages between 29-77 years old; all patient signed informed consent before to be treated. *Exclusion study criteria*: actual systemic antifungal therapy or antifungal 6 month before treatment, usage of local antifungal therapy, actual usage of nail coloring dyes, pregnancy and children under 12 years of age. Existence of concomitant nail disorders such as psoriasis of nail plate, lichen planus and atopic dermatitis. Total number of patients included in this study is 12, the clinical study is still currently on going, patients are regularly attending follow-up visits where they are investigated for clinical evaluation of the infection signs, microscopic exam and microbiological colture. So far 5 patients reached over 220 days of treatment and 3 ones over 150 days of treatment.

After nail sample collection was performed through cleansing of the nail area with water or saline solution to remove contaminants, for distal subungual onychomycosis, the infected nail was clipped proximally and the nail bed and underside of the nail plate were scraped with a 1-2 mm serrated curette. For proximal subungual onychomycosis, the normal surface of the nail plate was scarified with a surgical blade at the lunula and the white debris is collected with a sharp curette from the deeper portion of the nail plate and the proximal nail bed. The sampled material was divided into two portions: part for direct microscopy and part for culturing.

Direct seeding after scarification of the nail plate fragments was performed onto two plates of Sabouraud Gentamicin Chloramphenicol 2 agar, which is a selective isolation medium for yeast and mold, also etchings the plate surface to create an anaerobic environment. As dermatophytes can grow at different optimal temperatures one plate was incubated at 37 °C and another at 25 °C for at least 20 days to allow the microorganism growth. Subsequently the plates were analyzed under a microscope to identify the presence of dermatophyte, yeast or mold. Culture examination was independently executed.

Treatment efficacy was assessed by measuring the decrease in the area of the nail affected by the infection, or by complete infection eradication, confirmed by the microbiology data, compared to baseline (T0). Follow-ups were performed on 2, 3, 6, 7, 9 and 12 months. Patients were objectively evaluated for clearance of fungal infection, clinically by the podiatrist. Photographs were taken using the same camera setting, lighting.

During the clinical experimentation none of the treated patients reported phenomena of skin irritation, or any other toxic event due to composition administration.

### Results

### Types of diagnosed fungal infections

In the samples collected from patients of the treated group *Trichophyton mentagrophytes, Trichophyton rubrum, Aspergillus flavus*, *Candida* spp., saprophyte molds were detected, as expected mostly *Trichophyton mentagrophytes, Trichophyton rubrum.* Below are reported the exemplary case studies of 5 patients who were followed up for more than 220 days after the treatment starting.

### Case study 1

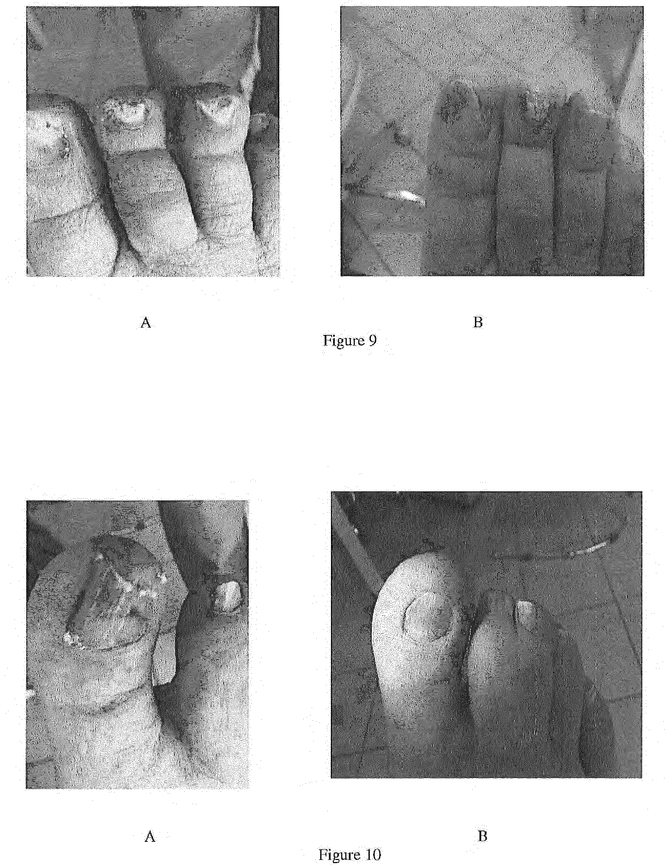

Patient identified with number 1 is a 73 years old female suffering of onychomycosis and unsuccessfully treated for one year with a topical antimycotic drug. At TO clinical analysis revealed three fingers of the right foot with onychomichosis, thickened nails, lateral onycholysis of the big toe, slight periungueal redness. Microscopic analysis of the nail samples showed the presence of saprophytic molds. According to the invention the patient received six weekly administrations of the composition based on carbamide peroxide and laponite in the solvent system as above described and the daily administration of the composition based on the association of *Melaleuca alternifolia* oil extract, mandelic acid, and α-Tocopherol polyethylene glycol succinate. A week after stopping the weekly administration the patient underwent clinical analysis by photographic evaluation compared to baseline images, microscopic exam and colture analysis. Microscopic analysis of the nail samples revealed the presence of *Trichophyton mentagrophytes* confirmed by microbiological colture data. The patient continued the treatment by the daily administration of the composition based on the association of *Melaleuca alternifolia* oil extract, mandelic acid, and α-Tocopherol polyethylene glycol succinate. Fifteen and 32 weeks after the starting of the treatment the patient underwent clinical analysis again, photographic evaluation compared to baseline images, microscopic exam and colture analysis was performed. Both microscopic exam and colture analysis data resulted to be negative. Photographic evaluation compared to baseline images is shown at Figure 9 revealed after 224 days of treatment an improvement in the appearance of the big toe and second finger nail and re-growing of the clear nail in the proximal part, especially in the big toe, and a complete healing of the third finger nail supporting the negative laboratory results.

### Case study 2

Patient identified with number 2 is a 65 years old male suffering of onychomycosis and so far unsuccessfully treated with a topical antimycotic drug. At TO clinical analysis revealed valgus big toe and traumatic nails, onychomichosis and thickened nails of the first three fingers of the right foot, onycholysis of the big toe. Fourth and fifth toes of the left foot appeared thick and mycotic as well. Microscopic analysis of the nail samples showed the presence of saprophytic molds. According to the invention the patient started the treatment by receiving six weekly administrations of the composition based on carbamide peroxide and laponite in the solvent system as described and the daily administration of the composition based on the association of *Melaleuca alternifolia* oil extract, mandelic acid, and α-Tocopherol polyethylene glycol succinate. A week after stopping the weekly administration the patient underwent clinical analysis by photographic evaluation compared to baseline images, microscopic exam and colture analysis. Microscopic analysis and microbiological colture confirmed the presence of saprophytic molds in the nail sample. In view of the positivity the treatment by three weekly additional administrations of the composition of carbamide peroxide and laponite and maintaining the usual daily administration of the composition based on the association of *Melaleuca alternifolia* oil extract, mandelic acid, and α-Tocopherol polyethylene glycol succinate was indicated. Fourteen and 32 weeks after the starting of the treatment the patient underwent clinical analysis by photographic evaluation compared to baseline images, microscopic exam and colture analysis. Both microscopic exam and colture analysis data resulted to be negative. Photographic evaluation compared to baseline images is shown at Figure 10 and revealed a remarkable improvement in the appearance of the finger, especially of the big toe and re-growing of the clear nail in the proximal part.

### Case study 3

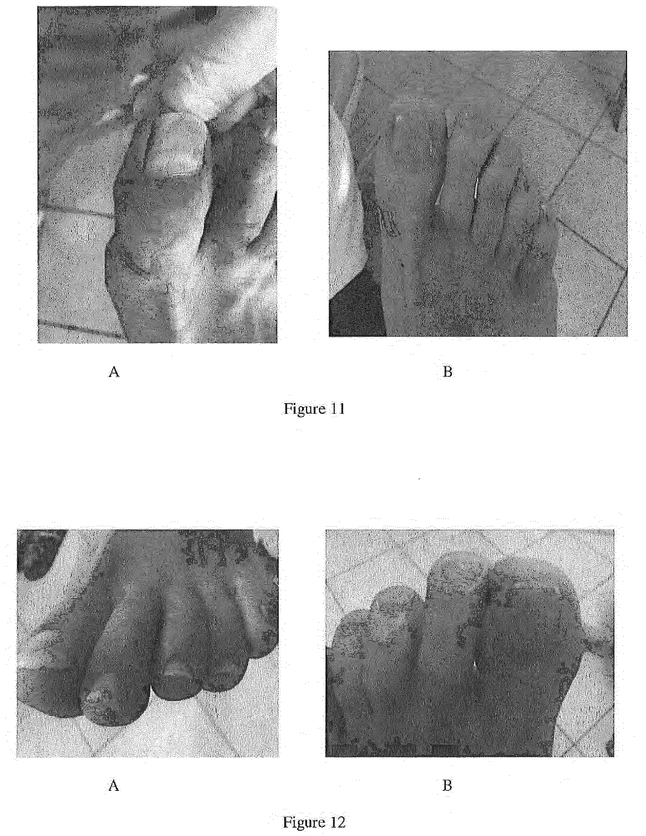

Patient identified with number 3 is a 45 years old male who has been suffering of onychomycosis for about three years. At TO clinical analysis revealed bilateral valgus big toe in both feet and traumatic nails, hammer toes or fingers in claffey, onychomichosis at big toe and fifth toe of the right foot, and onychomichosis at big toe, fourth and fifth toes in the left foot. Microscopic analysis of the nail samples showed the presence of saprophytic molds. According to the invention the patient started the treatment by six weekly administrations of the composition based on carbamide peroxide and laponite in the solvent system as described and the daily administration of the composition based on the association of *Melaleuca alternifolia* oil extract, mandelic acid, and α-Tocopherol polyethylene glycol succinate. A week after stopping the weekly administration the patient underwent clinical analysis by photographic evaluation compared to baseline images, microscopic exam and colture analysis. Microscopic analysis of the nail and microbiological colture confirmed the presence of saprophytic molds. The patient continued the treatment by three additional weekly administrations of the composition of carbamide peroxide and laponite and keeping on with the usual daily administration of the composition based on the association of *Melaleuca alternifolia* oil extract, mandelic acid, and α-Tocopherol polyethylene glycol succinate. Fourteen and 32 weeks after the starting of the treatment the patient underwent clinical analysis by photographic evaluation compared to baseline images, microscopic exam and colture analysis. Both microscopic exam and colture analysis data resulted to be negative. More than 220 days after the start of the treatment with the association of composition according to the invention photographic evaluation of the pictures taken during the last follow up visit compared to baseline images confirmed the absence of clinical signs of fungal infection and the on-going re-growing of the nail (Fig. 11).

### Case study 4

Patient identified with number 4 is a 57 years old female. At TO clinical analysis revealed onychomychosis signs in all toes of both feet. Microscopic analysis of the nail samples showed the presence of micro-spores from *Trichophyton mentagrophytes,* data was confirmed by microbiologic colture analysis. According to the invention the patient received six weekly administrations of the composition based on carbamide peroxide and laponite as described and the daily administration of the composition based on the association of *Melaleuca alternifolia* oil extract, mandelic acid, and α-Tocopherol polyethylene glycol succinate. One week after stopping the weekly administration the patient underwent clinical analysis by photographic evaluation compared to baseline images, microscopic exam and colture analysis. Microscopic analysis of the nail and microbiological colture have not shown presence of any dermatophyte, non-dermatophyte fungus, yeast or mold. The patient continued the treatment by the usual daily administration of the composition based on the association of *Melaleuca alternifolia* oil extract, mandelic acid, and α-Tocopherol polyethylene glycol succinate. Fourteen and 32 weeks after the starting of the treatment the patient underwent clinical analysis by photographic evaluation compared to baseline images, microscopic exam and colture analysis. Both microscopic exam and colture analysis data resulted to be negative. Photographic evaluation compared to baseline images is shown at Figure 12 and revealed a remarkable improvement in the appearance of the finger, especially of the big toe and proximal re-growing of clear nail.

### Case study 5

A 52 year old male patient presenting dystrophic and thick finger nails, bilateral valgus big toe, onychomychosis at big toe, second toe of right foot, onychomychosis at big toe, second, fourth and fifth toe of left foot. Microscopic analysis of the nail samples showed the presence of micro spores from *Trichophyton rubrum,* data was confirmed by microbiologic colture analysis. According to the invention the patient received six weekly administrations of the composition based on carbamide peroxide and laponite in the solvent system as described and the daily administration of the composition based on the association of *Melaleuca alternifolia* oil extract, mandelic acid, and α-Tocopherol polyethylene glycol succinate. One week after stopping the weekly administration the patient underwent clinical analysis by photographic evaluation compared to baseline images, microscopic exam and colture analysis. Microscopic analysis of the nail revealed in the nail sample the presence of *Aspergillus flavus* hyphae which however was not confirmed by microbiological colture exam. The patient continued the treatment by three additional weekly administrations of the composition of carbamide peroxide and laponite and by keeping on with the usual daily administration of the composition based on the association of *Melaleuca alternifolia* oil extract, mandelic acid, and α-Tocopherol polyethylene glycol succinate. Fourteen weeks after the starting of the treatment the patient underwent clinical analysis by photographic evaluation compared to baseline images, microscopic exam and colture analysis. Both microscopic and microbiological results were negative. The patient continued the treatment with the composition of *Melaleuca alternifolia* oil extract, mandelic acid, and vitamin E derivate.

At the follow up visit 32 weeks after the starting of the treatment microscopic analysis of the nail and microbiological colture exam revealed in the nail sample the presence of *Trichophyton mentagrophytes* spores.

Table 17 summarizes the results of the clinical experimentation so far obtained.

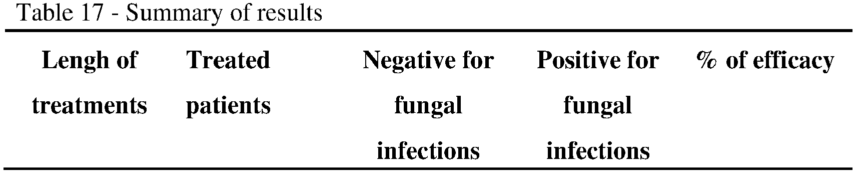

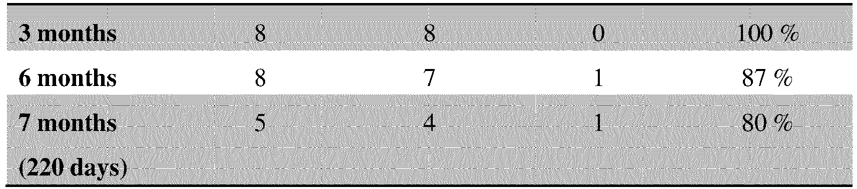

Based on the obtained results in this still limited group of patients enrolled from two centers (a further clinical study is currently on going on a larger group of patients), out of 8 patients having history of recurrent onychomychosis with clinically and microbiologically proven infection due to dermatophytes and non-dermatophyte fungi, who reached a more than 220 days follow-up period, three months after the starting of the treatment all treated patients (100%) resulted to be cleared of fungal infection. Six months after the starting of the treatment only one patient out of the tested 8 resulted to be positive for fungal infection, thereby the treatment showed 87% efficacy rate in eradicating the fungal infection and maintaining the site clear from dermatophytes and non-dermatophyte fungi or molds. Out of the five patients who received the prolonged treatment up to more than 220 days, only one resulted to be positive for fungal infection (the same patient who resulted positive at six months follow up visit).

Therefore, although the clinical study is still on-going and the follow up period not completed, so far the treatment according to the invention based on the administration of the two compositions as described demonstrated to enable the clearance of fungal infection for more than seven months in 80 % of treated patients without causing any adverse effect correlated with the administration of the compositions for a such prolonged time period.

The herein described results clearly demonstrated that the topical administration of the association of the two compositions enable to maintain in the time the positive effect sorted by the administration of the composition comprising: carbamide peroxide in a variable amount ranging between 22 and 3% by weight of the composition, laponite powder in an amount ranging from 18 to 2% by weight of the composition, in a solvent which is well suited for eradicating nail fungal infection. In fact, a previous clinical study performed by the Applicants demonstrated that the weekly application of such composition once for 3 months, sometimes up to 6 months, produced mycological and clinical cure in more than 85 % of patients with onychomycosis, even severe. However, several weeks after stopping the composition administration about 80% of the treated patients, particularly those having a history of recurrent fungal infection of the nails, reverted to show fungal infection clinical signs.

Instead, the association of the two compositions determines a synergistic effect wherein the composition based on the association of *Melaleuca alternifolia* oil extract, mandelic acid, and α-Tocopherol polyethylene glycol succinate stabilizes and maintain in the time the positive effect of the composition weekly administrated for 6-9 weeks at the beginning of the treatment, exerting a prophylactic effect towards the development of new infective events. This result is particularly important for those patients predisposed to recurrent onychomychosis. For sure the follow-up period has to be prolonged and the fungal infection establishing monitored for a longer period of time, however these preliminary results are encouraging when compared with prior art data showing that amorolfine topically administrated twice a week provides a prophylaxy able to retard the recurrent event of about 200 days [Sigurgeisson B. et al., Efficacy of amorolfine nail lacquer for the prophylaxis of the onychomychosis over three years. J Eur Acad Dermatol Venereol 2010; 24: 910-915]. On the other hand the treatment according to the present invention is based on the sinergistic effect determined by two compositions, simple to be applied, not comprising compounds such as amorolfine, econazolo, clotrimazolo, griseofulvina, ketoconazolo, miconazolo, nistatina, which are not without side effects, nail discoloration and fragility. [Rigopoulos D. "Discoloration of the nail plate due to the misuse of amorolfine 5% nail lacquer". Acta Derm Venereol (1996) 76: 83-4].

### Conclusion

The present invention has shown to overcome the technical problems still present in the topical therapies used against the fungal nail infection. While the various types of topical treatment of the state of the art, in the form of nail polish, gel, ointment, cream or solution have so far shown limited effectiveness, useful only for the remission of lesions not too extensive, but not able to prevent the onset of recurring infections, the present invention combines in itself the advantage of not causing side effects typical of a topical treatment, characterized by a high degree of effectiveness even in those cases resistant to treatments of the state of the art and destined to face a new infectious episode.

## Claims

1. A composition **characterized in that** it comprises the association of:
a) *Melaleuca alternifolia* oil extract,
b) mandelic acid,
c) α-Tocopherol polyethylene glycol succinate,
wherein said three components of the association respectively are in weight ratio: tea tree oil/mandelic acid/Vit. E TPGS of 12/0.5/0.5.

2. The composition according to claim 1 further comprising: carrier agents, solvents, essential oils, fragrances and perfumes, preservatives, emollients and moisturizing agents, antioxidants, vitamins, surfactants, suspending agents, binders, film-forming and viscosity agents, thickeners, chelating agents, buffering agents pharmaceutically accepted.

3. The composition according to claim 2 wherein carrier agents, solvents, essential oils, fragrances and perfumes, preservatives, emollients and moisturizing agents, antioxidant agents, vitamins, surfactants, suspending agents, binders, film-forming and viscosity agents, thickeners, chelating agents, buffering agents, pharmaceutically accepted are: lavender oil, bergamot oil, eucalyptus oil, geraniol, oil extracted from the germ and the inner film of rice, hydrolyzed wheat protein, lecithin, ascorbyl palmitate, carnosine, lipoic acid, propylene glycol, xanthan gum, hydroxyethyl acrylate/ sodium acryloyldimethyl taurate copolymer.

4. The composition according to claims 1-3 having the formulation:
| | | |
|---|---|---|
| Oil extract of *Melaleuca alternifolia* | 12% | w/w |
| Mandelic acid | 0.5% | w/w |
| Vit. E TPGS | 0.5% | w/w |
| *Lavandula angustifolia* oil | 6.0% | w/w |
| Propylene glycol | 2.5% | w/w |
| Xanthan gum | 2.0% | w/w |
| Linalool | 1.5% | w/w |
| Hydroxyethyl acrylate/sodium acryloyl dimethyl taurate copolymer | 1.11% | w/w |
| Squalane | 0.765% | w/w |
| Carnosine | 0.5% | w/w |
| Lipoic acid | 0.5% | w/w |
| Limonene | 0.204% | w/w |
| Polysorbate 60 | 0.165% | w/w |
| Tetrasodium glutamate diacetate | 0.094% | w/w |
| Geraniol | 0.06% | w/w |
| Sorbitan isostearate | 0.045% | w/w |
| Citronellol | 0.006% | w/w |
| Coumarine | 0.006% | w/w |
| Deionized water | qs to 100. | |

5. The composition according to claims 1-3 having the formulation:
| | | |
|---|---|---|
| Oil extract of *Melaleuca alternifolia* | 12% | w/w |
| Mandelic acid | 0.5% | w/w |
| Vit. E TPGS | 0.5% | w/w |
| *Citrus bergamia* essential oil | 6.0% | w/w |
| Caprylic/capric triglyceride | 5.425% | w/w |
| Ethylhexyl palmitate | 5.037% | w/w |
| Glycerin | 4.65% | w/w |
| Cetostearyl alcohol | 3.83% | w/w |
| Limonene | 2.84% | w/w |
| Steareth-2 | 2.8% | w/w |
| Steareth-20 | 1.4% | w/w |
| Propylene glycol | 2.5% | w/w |
| Tocopheryl acetate | 1.55% | w/w |
| Xanthan gum | 2.0% | w/w |
| Linalool | 0.9% | w/w |
| Dimethicone | 0.62% | w/w |
| Benzyl alcohol | 0.5% | w/w |
| Carnosine | 0.5% | w/w |
| Lipoic acid | 0.5% | w/w |
| Oil extracted from the germ and the inner film of rice | 0.387% | w/w |
| Protein Hydrolyzed Wheat | 0.11% | w/w |
| Sodium dehydroacetate | 0.11% | w/w |
| Lecithin | 0.087 | w/w |
| Tetrasodium glutamate diacetate | 0.05% | w/w |
| Dehydroacetic acid | 0.032% | w/w |
| Tocopherol | 0.014% | w/w |
| Ascorbyl palmitate | 0.010% | w/w |
| Citric Acid | 0.003% | w/w |
| Deionized water | qs to 100. | |

6. The composition according to any one of the preceding claims in a form suitable for topical administration.

7. The composition comprising the association of:
a) *Melaleuca alternifolia* oil extract,
b) mandelic acid,
c) α-Tocopherol polyethylene glycol succinate,
wherein said three components of the association respectively are in weight ratio: tea tree oil/mandelic acid/Vit. E TPGS of 12/0.5/0.5, as defined in claims 1 to 6, for the use in the treatment of onychomycosis.

8. The composition for the use according to claim 7 wherein an effective amount of the composition is applied on the nail affected by fungal infection and surrounding skin, once a day, generally in the evening before bedtime, after cleansing, and rub until completely absorbed.

9. An association of the composition as defined in claims 1 to 6 and a composition comprising:
a) carbamide peroxide in an amount ranging from 22 to 3% by weight of the composition,
b) laponite powder in an amount ranging from 18 to 2% by weight of the composition,
c) solvent system,
wherein the solvent system is: deionized water, propylene glycol, a mixture of deionized water / propylene glycol (90/10 - 10-90 v/v).

10. The association according to claim 9 wherein the composition comprising carbamide peroxide, laponite and the solvent system further comprises an inhibitor of the catalase enzyme.

11. The association according to claim 10 wherein the inhibitor of catalase is hydroxylamine or 3-amino-1,2,4-triazolo.

12. An association as defined in claims 9-11 for the use in the treatment of recurrent onychomycosis.

13. The association for the use according to claim 12, wherein a therapeutically effective amount of the composition comprising carbamide peroxide and laponite in a solvent system sufficient to cover the region concerned by infection is applied once a week for a time varying from 10 to 30 minutes for at least four weeks, after nail cut and a thorough cleaning of the nail bed to remove the most of all nail fragments, in combination with the daily topical application of a therapeutically effective amount of the composition comprising the association of *Melaleuca alternifolia* oil extract, mandelic acid and Vit. E TPGS, usually before bedtime, after cleansing, said composition being rubbed on the skin and nail until fully absorbed.

14. The association for the use according to claim 13 wherein the daily topical application of the composition comprising the association of *Melaleuca alternifolia* oil extract, mandelic acid and Vit. E TPGS is repeated until needed or by complete infection healing.

15. The association for the use according to claims 12 to 14 comprising the steps of:
a) nail cutting and deep cleansing of the nail bed to remove nail fragments;
b) weekly topical application of the composition in the form of gels prepared in the following manner :
i) preparation of gel by dissolving the powder mixture of carbamide peroxide and laponite XGL in the solvent, and mixing thoroughly with the help, if necessary, of a spatula;
ii) application of the gel obtained in i) on the nail to be treated;
iii) removal of the gel;
iv) rinsing with plenty of saline or water, and in conjunction
c) daily topical application of the composition comprising the association of *Melaleuca alternifolia* oil extract, mandelic acid and Vit. E TPGS.

16. A kit comprising:
(a) a sachet type container of moisture and air impermeable material containing the amount of carbamide peroxide necessary for an administration;
(b) a sachet type container of moisture and air impermeable material containing the amount of laponite XGL, and possibly inhibitor of catalase, necessary for an administration;
(c) a vial type container containing the amount of solvent system, selected from deionized water, propylene glycol and the mixture of deionized water/propylene glycol (90/10 - 10-90 v/v), required for an administration;
(d) a small spatula or stirrer in an inert material to facilitate the mixing of the contents of the two sachets;
(e) a container containing the topical composition comprising the association of *Melaleuca alternifolia* oil extract, mandelic acid and Vit. E TPGS and pharmaceutically accepted agents;
(f) leaflet with instructions for the use.

## Patentansprüche

1. Zusammensetzung, **dadurch gekennzeichnet, dass** sie die Assoziation umfasst von:
a) *Melaleuca alternifolia* Ölextrakt,
b) Mandelsäure,
c) α-Tocopherol-Polyethylenglycolsuccinat,
wobei die drei Komponenten der Assoziation ein Gewichtsverhältnis von: Teebaumöl/Mandelsäure/Vit. E TPGS von 12/0,5/0,5 aufweisen.

2. Zusammensetzung nach Anspruch 1, weiter umfassend: Trägermittel, Lösemittel, essentielle Öle, Duftstoffe und Parfüms, Konservierungsmittel, Weichmacher und Befeuchtungsmittel, Antioxidantien, Vitamine, Tenside, Suspendierungsmittel, Bindemittel, Filmbildungs- und Viskositätsmittel, Verdickungsmittel, Chelatbildner, Puffermittel, die pharmazeutisch annehmbar sind.

3. Zusammensetzung nach Anspruch 2 wobei Trägermittel, Lösemittel, essentielle Öle, Duftstoffe und Parfüms, Konservierungsmittel, Weichmacher und Befeuchtungsmittel, Antioxidantien, Vitamine, Tenside, Suspendierungsmittel, Bindemittel, Filmbildungs- und Viskositätsmittel, Verdickungsmittel, Chelatbildner, Puffermittel, die pharmazeutisch annehmbar sind, sind: Lavendelöl, Bergamottöl, Eukalyptusöl, Geraniol, Öl, das aus dem Keim und dem Innenhäutchen von Reis extrahiert ist, Weizenproteinhydrolysat, Lecithin, Ascorbylpalmitat, Carnosin, Liponsäure, Propylenglycol, Xanthangummi, Hydroxyethylacrylat/ Natriumacryloyldimethyltaurat-Copolymer.

4. Zusammensetzung nach Anspruch 1 - 3 mit der Formulierung:
| | | |
|---|---|---|
| Ölextrakt von *Melaleuca alternifolia* | 12 % | w/w |
| Mandelsäure | 0,5 % | w/w |
| Vit. E TPGS | 0,5 % | w/w |
| *Lavandula angustifolia* Öl | 6,0 % | w/w |
| Propylenglycol | 2,5 % | w/w |
| Xanthangummi | 2,0 % | w/w |
| Linalool | 1,5 % | w/w |
| Hydroxyethylacrylat/Natriumacryloyldimethyltaurat-Copolymer | 1,11 % | w/w |
| Squalan | 0,765 % | w/w |
| Carnosin | 0,5 % | w/w |
| Liponsäure | 0,5 % | w/w |
| Limonen | 0,204 % | w/w |
| Polysorbat 60 | 0,165 % | w/w |
| Tetranatriumglutamatdiacetat | 0,094 % | w/w |
| Geraniol | 0,06 % | w/w |
| Sorbitanisostearat | 0,045 % | w/w |
| Citronellol | 0,006 % | w/w |
| Cumarin | 0,006 % | w/w |
| Entionisiertes Wasser | qs auf 100. | |

5. Zusammensetzung nach Anspruchs 1 - 3 mit der Formulierung:
| | | |
|---|---|---|
| Ölextrakt von Melaleuca alternifolia | 12 % | w/w |
| Mandelsäure | 0,5 % | w/w |
| Vit. E TPGS | 0,5 % | w/w |
| *Citrus bergamia* essentielles Öl | 6,0 % | w/w |
| Capryl-/Caprin-Triglycerid | 5,425 % | w/w |
| Ethylhexylpalmitat | 5,037 % | w/w |
| Glycerin | 4,65 % | w/w |
| Cetostearylalkohol | 3,83 % | w/w |
| Limonen | 2,84 % | w/w |
| Steareth-2 | 2,8 % | w/w |
| Steareth-20 | 1,4 % | w/w |
| Propylenglycol | 2,5 % | w/w |
| Tocopherylacetat | 1,55 % | w/w |
| Xanthangummi | 2,0 % | w/w |
| Linalool | 0, 9 % | w/w |
| Dimethicon | 0,62 % | w/w |
| Benzylalkohol | 0,5 % | w/w |
| Carnosin | 0,5 % | w/w |
| Liponsäure | 0,5 % | w/w |
| Öle, extrahiert aus dem Keim und dem | 0,387 % | w/w |
| Innenhäutchen von Reis Weizenproteinhydrolysat | 0,11 % | w/w |
| Natriumdehydroacetat | 0,11 % | w/w |
| Lecithin | 0,087 | w/w |
| Tetranatriumglutamatdiacetat | 0,05 % | w/w |
| Dehydroessigsäure | 0,032 % | w/w |
| Tocopherol | 0,014 % | w/w |
| Ascorbylpalmitat | 0,010 % | w/w |
| Zitronensäure | 0,003 % | w/w |
| Entionisiertes Wasser | qs auf 100. | |

6. Zusammensetzung nach einem der vorstehenden Ansprüche in einer Form, die zur topischen Verabreichung geeignet ist.

7. Zusammensetzung, umfassend die Assoziation von:
a) *Melaleuca alternifolia* Ölextrakt,
b) Mandelsäure,
c) α-Tocopherol-Polyethylenglycolsuccinat,
wobei die drei Komponenten der Assoziation ein Gewichtsverhältnis von: Teebaumöl/Mandelsäure/Vit. E TPGS von 12/0,5/0,5 aufweisen, wie in Ansprüchen 1 bis 6 definiert, für die Verwendung in der Behandlung von Onychomykose.

8. Zusammensetzung für die Verwendung nach Anspruch 7, wobei eine wirksame Menge der Zusammensetzung auf den Nagel, der von einer Pilzinfektion befallen ist, und auf umgebende Haut einmal täglich aufgetragen wird, im Allgemeinen am Abend vor dem Schlafengehen, nach Reinigung, und eingerieben wird, bis sie vollständig absorbiert ist.

9. Assoziation der Zusammensetzung wie in Ansprüchen 1 bis 6 definiert, und einer Zusammensetzung, umfassend:
a) Carbamidperoxid in einer Menge im Bereich von 22 bis 3 Gewichtsprozent der Zusammensetzung,
b) Laponitpulver in einer Menge im Bereich von 18 bis 2 Gewichtsprozent der Zusammensetzung,
c) Lösemittelsystem,
wobei das Lösemittelsystem ist: entionisiertes Wasser, Propylenglycol, ein Gemisch aus entionisiertem Wasser/Propylenglycol (90/10 - 10-90 v/v).

10. Assoziation nach Anspruch 9, wobei die Zusammensetzung, die Carbamidperoxid, Laponit und das Lösemittelsystem umfasst, weiter einen Inhibitor des Katalase-Enzyms umfasst.

11. Assoziation nach Anspruch 10, wobei der Inhibitor der Katalase Hydroxylamin oder 3-Amino-1,2,4-triazolo ist.

12. Assoziation wie in Ansprüchen 9 - 11 definiert, für die Verwendung in der Behandlung wiederkehrender Onchomykose.

13. Assoziation für die Verwendung nach Anspruch 12, wobei eine therapeutisch wirksame Menge der Zusammensetzung, die Carbamidperoxid und Laponit in einem Lösemittelsystem umfasst, die ausreichend ist, das Gebiet, das von Infektion befallen ist, zu bedecken, einmal wöchentlich für eine Zeit, die von 10 bis 30 Minuten variiert, über mindestens vier Wochen nach Nagelschneiden und gründlicher Reinigung des Nagelbetts, um die meisten aller Nagelfragmente zu entfernen, in Kombination mit der täglichen topischen Anwendung einer therapeutisch wirksamen Menge der Zusammensetzung, die die Assoziation von *Melaleuca alternifolia* Ölextrakt, Mandelsäure und Vit. E TPGS umfasst, üblicherweise vor dem Schlafengehen, nach Reinigung aufgetragen wird, wobei die Zusammensetzung auf der Haut und dem Nagel eingerieben wird, bis sie vollständig absorbiert ist.

14. Assoziation für die Verwendung nach Anspruch 13, wobei die tägliche topische Anwendung der Zusammensetzung, die die Assoziation aus *Melaleuca alternifolia* Ölextrakt, Mandelsäure und Vit. E TPGS umfasst, bis zur vollständigen Heilung der Infektion wiederholt wird.

15. Assoziation für die Verwendung nach Ansprüchen 12 bis 14, umfassend die Schritte:
a) Nagelschneiden und Tiefenreinigung des Nagelbetts, um Nagelfragmente zu entfernen;
b) wöchentliche topische Anwendung der Zusammensetzung in der Form von Gelen, die auf folgende Weise zubereitet werden:
i) Zubereitung von Gel durch Auflösen des Pulvergemisches aus Carbamidperoxid und Laponit XGL in dem Lösemittel und gründliches Mischen, falls notwendig mit der Hilfe einer Spachtel;
ii) Auftragen des in i) erhaltenen Gels auf den zu behandelnden Nagel;
iii) Entfernen des Gels;
iv) Spülen mit viel Kochsalzlösung oder Wasser und in Verbindung
c) tägliche topische Anwendung der Zusammensetzung, die die Assoziation von *Melaleuca alternifolia* Ölextrakt, Mandelsäure und Vit. E TPGS umfasst.

16. Kit, umfassend:
(a) einen säckchenartigen Behälter aus feuchtigkeits- und luftundurchlässigem Material, der die Menge an Carbamidperoxid enthält, die für eine Verabreichung notwendig ist;
(b) einen säckchenartigen Behälter aus feuchtigkeits- und luftundurchlässigem Material, der die Menge an Laponit XGL und möglicherweise eines Katalase-Inhibitors enthält, die für eine Verabreichung notwendig ist;
(c) einen ampullenförmigen Behälter, der die Menge an Lösemittelsystem enthält, ausgewählt aus entionisiertem Wasser, Propylenglycol und dem Gemisch aus entionisiertem Wasser/Propylenglycol (90/10 - 10-90 v/v), die für eine Verabreichung notwendig ist;
(d) eine kleine Spachtel oder einen Rührer in einem inerten Material, um das Mischen der Inhaltsstoffe der zwei Säckchen zu erleichtern;
(e) einen Behälter, der die topische Zusammensetzung, die die Assoziation aus *Melaleuca alternifolia* Ölextrakt, Mandelsäure und Vit. E TPGS umfasst, und pharmazeutisch annehmbare Mittel enthält;
(f) Beipackzettel mit Anweisungen für die Verwendung.

## Revendications

1. Composition **caractérisée en ce qu'**elle comprend l'association :
a) d'un extrait d'huile de *Melaleuca alternifolia,*
b) d'acide mandélique,
c) d'un succinate de polyéthylèneglycol α-tocophérol,
dans laquelle lesdits trois composants de l'association sont respectivement dans un rapport pondéral : huile de théier/acide mandélique/Vit. E TPGS de 12/0,5/0,5.

2. Composition selon la revendication 1 comprenant en outre : des agents de support, des solvants, des huiles essentielles, des fragrances et des parfums, des conservateurs, des émollients et des agents hydratants, des antioxydants, des vitamines, des tensioactifs, des agents de mise en suspension, des liants, des agents filmogènes et de viscosité, des épaississants, des agents de chélation, des agents tampons acceptés en pharmacie.

3. Composition selon la revendication 2, dans laquelle les agents de support, les solvants, les huiles essentielles, les fragrances et les parfums, les conservateurs, les émollients et les agents hydratants, les agents antioxydants, les vitamines, les tensioactifs, les agents de mise en suspension, les liants, les agents filmogènes et de viscosité, les épaississants, les agents de chélation, les agents tampons acceptés en pharmacie sont : l'huile de lavande, l'huile de bergamote, l'huile d'eucalyptus, le géraniol, une huile extraite du germe et du film interne du riz, une protéine de blé hydrolysée, la lécithine, le palmitate d'ascorbyle, la carnosine, l'acide lipoïque, le propylène glycol, la gomme de xanthane, un copolymère d'acrylate d'hydroxyéthyle/ acryloyldiméthyltaurate de sodium.

4. Composition selon les revendications 1 à 3 ayant la formule :
| | |
|---|---|
| Extrait d'huile de *Melaleuca alternifolia* | 12 % en poids/poids |
| Acide mandélique | 0,5 % en poids/poids |
| Vit. E TPGS | 0,5 % en poids/poids |
| Huile de *Lavandula angustifolia* | 6,0 % en poids/poids |
| Propylène glycol | 2,5 % en poids/poids |
| Gomme de xanthane | 2,0 % en poids/poids |
| Linalool | 1,5 % en poids/poids |
| Copolymère d'acrylate d'hydroxyéthyle/acryloyldiméthyltaurate de sodium | 1,11 % en poids/poids |
| Squalane | 0,765 % en poids/poids |
| Carnosine | 0,5 % en poids/poids |
| Acide lipoïque | 0,5 % en poids/poids |
| Limonène | 0,204 % en poids/poids |
| Polysorbate 60 | 0,165 % en poids/poids |
| Diacétate de glutamate tétrasodique | 0,094 % en poids/poids |
| Géraniol | 0,06 % en poids/poids |
| Isostéarate de sorbitane | 0,045 % en poids/poids |
| Citronellol | 0,006 % en poids/poids |
| Coumarine | 0,006 % en poids/poids |
| Eau déionisée | qs jusqu'à 100 |

5. Composition selon les revendications 1 à 3 ayant la formule :
| | |
|---|---|
| Extrait d'huile de *Melaleuca alternifolia* | 12 % en poids/poids |
| Acide mandélique | 0,5 % en poids/poids |
| Vit. E TPGS | 0,5 % en poids/poids |
| Huile essentielle de *Citrus bergamia* | 6,0 % en poids/poids |
| Triglycéride caprylique/caprique | 5,425 % en poids/poids |
| Palmitate d'éthylhexyle | 5,037 % en poids/poids |
| Glycérine | 4,65 % en poids/poids |
| Alcool cétostéarylique | 3,83 % en poids/poids |
| Limonène | 2,84 % en poids/poids |
| Stéareth-2 | 2,8 % en poids/poids |
| Stéareth-20 | 1,4 % en poids/poids |
| Propylène glycol | 2,5 % en poids/poids |
| Acétate de tocophéryle | 1,55 % en poids/poids |
| Gomme de xanthane | 2,0 % en poids/poids |
| Linalool | 0,9 % en poids/poids |
| Diméthicone | 0,62 % en poids/poids |
| Alcool benzylique | 0,5 % en poids/poids |
| Carnosine | 0,5 % en poids/poids |
| Acide lipoïque | 0,5 % en poids/poids |
| Huile extraite du germe et du film interne du riz | 0,387 % en poids/poids |
| Protéine de blé hydrolysée | 0,11 % en poids/poids |
| Déhydroacétate de sodium | 0,11 % en poids/poids |
| Lécithine | 0,087 en poids/poids |
| Diacétate de glutamate tétrasodique | 0,05 % en poids/poids |
| Acide déhydroacétique | 0,032 % en poids/poids |
| Tocophérol | 0,014 % en poids/poids |
| Palmitate d'ascorbyle | 0,010 % en poids/poids |
| Acide citrique | 0,003 % en poids/poids |
| Eau déionisée | qs jusqu'à 100 |

6. Composition selon l'une quelconque des revendications précédentes sous une forme appropriée pour une administration topique.

7. Composition comprenant l'association :
a) d'un extrait d'huile de *Melaleuca alternifolia,*
b) d'acide mandélique,
c) d'un succinate de polyéthylèneglycol α-tocophérol,
dans laquelle lesdits trois composants de l'association sont respectivement dans un rapport pondéral : huile de théier/acide mandélique/Vit. E TPGS de 12/0,5/0,5, comme défini dans les revendications 1 à 6, pour utilisation dans le traitement d'une onychomycose.

8. Composition pour utilisation selon la revendication 7, dans laquelle une quantité efficace de la composition est appliquée sur l'ongle affecté par une infection fongique et la peau environnante, une fois par jour, généralement le soir avant le coucher, après nettoyage, et frotter jusqu'à absorption complète.

9. Association de la composition telle que définie dans les revendications 1 à 6 et d'une composition comprenant :
a) du peroxyde de carbamide en une quantité allant de 22 à 3 % en poids de la composition,
b) une poudre de laponite en une quantité allant de 18 à 2 % en poids de la composition,
c) un système de solvant,
dans laquelle le système de solvant est : l'eau désionisée, le propylène glycol, un mélange eau désionisée/propylène glycol (90/10 à 10-90 en volume/volume).

10. Association selon la revendication 9, dans laquelle la composition comprenant du peroxyde de carbamide, de la laponite et le système de solvant comprend en outre un inhibiteur de l'enzyme catalase.

11. Association selon la revendication 10, dans laquelle l'inhibiteur de catalase est l'hydroxylamine ou le 3-amino-1,2,4-triazolo.

12. Association telle que définie dans les revendications 9 à 11 pour utilisation dans le traitement d'une onychomycose récurrente.

13. Association pour utilisation selon la revendication 12, dans laquelle une quantité thérapeutiquement efficace de la composition comprenant du peroxyde de carbamide et de la laponite dans un système de solvant suffisant pour couvrir la région concernée par l'infection est appliquée une fois par semaine pendant une durée variant de 10 à 30 minutes pendant au moins quatre semaines, après coupe de l'ongle et nettoyage en profondeur du lit de l'ongle pour éliminer la majeure partie des fragments d'ongle, en combinaison avec l'application topique quotidienne d'une quantité thérapeutiquement efficace de la composition comprenant l'association d'extrait d'huile de *Melaleuca alternifolia,* d'acide mandélique et de Vit. E TPGS, habituellement avant le coucher, après nettoyage, ladite composition étant frottée sur la peau et l'ongle jusqu'à absorption complète.

14. Association pour utilisation selon la revendication 13, dans laquelle l'application topique quotidienne de la composition comprenant l'association d'extrait d'huile de *Melaleuca alternifolia,* d'acide mandélique et de Vit. E TPGS est répétée tant que nécessaire ou jusqu'à guérison complète de l'infection.

15. Association pour utilisation selon les revendications 12 à 14, comprenant les étapes suivantes :
a) la coupe de l'ongle et le nettoyage en profondeur du lit de l'ongle pour éliminer les fragments d'ongle ;
b) l'application topique hebdomadaire de la composition sous la forme de gels préparés de la manière suivante :
i) préparation du gel en dissolvant le mélange pulvérulent de peroxyde de carbamide et de laponite XGL dans le solvant, et en mélangeant parfaitement à l'aide, si nécessaire, d'une spatule ;
ii) application du gel obtenu en i) sur l'ongle à traiter ;
iii) élimination du gel ;
iv) rinçage abondant avec une solution physiologique salée ou de l'eau, et en même temps
c) l'application topique quotidienne de la composition comprenant l'association d'extrait d'huile de *Melaleuca alternifolia*, d'acide mandélique et de Vit. E TPGS.

16. Kit comprenant :
(a) un récipient de type sachet en matériau imperméable à l'humidité et à l'air contenant la quantité de peroxyde de carbamide nécessaire pour une administration ;
(b) un récipient de type sachet en matériau imperméable à l'humidité et à l'air contenant la quantité de laponite XGL, et éventuellement un inhibiteur de catalase, nécessaire pour une administration ;
(c) un récipient de type flacon contenant la quantité de système de solvant, sélectionné parmi l'eau désionisée, le propylène glycol et le mélange eau désionisée/propylène glycol (90/10 à 10-90 en volume/volume), nécessaire pour une administration ;
(d) une petite spatule ou un agitateur en un matériau inerte pour faciliter le mélange des contenus des deux sachets ;
(e) un récipient contenant la composition topique comprenant l'association d'extrait d'huile de *Melaleuca alternifolia,* d'acide mandélique et de Vit. E TPGS et des agents acceptés en pharmacie ;
(f) une notice avec des instructions pour l'utilisation.
